# DEMANDE DE BREVET EUROPEEN

(11) **EP 0 607 091 A1**
(43) Date de publication de la demande: **20.07.1994**
(21) Numéro de dépôt: 94400094.2
(22) Date de dépôt: 14.01.1994
(51) Int. Cl.: C12Q 1/42, C07D 493/04

(54) **Méthode de dosage des furocoumarines, et plus particulièrement du psoralène et de ses dérivés, composés et kits pour la mise en oeuvre de cette méthode**

(30) Priorité: 15.01.1993 FR 9300353
(71) Demandeur: Goupil, Jean Jacques, F-92000 Boulogne (FR)
(72) Inventeur: Goupil, Jean Jacques, F-92000 Boulogne (FR)
(74) Mandataire: Petit, Hélène

(57) **Abrégé**

La présente invention a pour objet une méthode de dosage des furocoumarines réalisée à l'aide d'anticorps anti-furocoumarines obtenus par immunisation d'animaux avec des complexes furocoumarines/molécules porteuses antigéniques.

L'invention vise également les réactifs et les kits contenant ces réactifs pour la mise en oeuvre de cette méthode de dosage.

## Description

La présente invention a pour objet une méthode de dosage des furocoumarines, et plus particulièrement du psoralène et de ses dérivés. Elle vise également les réactifs et les kits (ou trousses) contenant ces réactifs pour la mise en oeuvre de cette méthode de dosage.

Les furocoumarines, et plus particulièrement les dérivés du psoralène, sont utilisées dans le cadre du traitement de nombreuses pathologies. A titre illustratif le 5-methoxypsoralène (5-MOP) est utilisé en puvathérapie contre le psoriasis, ou encore en tant qu'antidépresseur ou anticancéreux.

Il est particulièrement intéressant de connaître avec précision le dosage de ces dérivés du psoralène dans l'organisme d'un individu traité avec ces dérivés, notamment dans le cadre de l'utilisation du 5-MOP en puvathérapie, afin d'ajuster en cours de traitement le dosage du 5-MOP en fonction de la quantité de radiations ultraviolets A (UVA) reçue.

Cependant les méthodes actuelles de dosage des furocoumarines sont essentiellement représentées par des techniques de chromatographie liquide haute pression (HPLC) lourdes à mettre en oeuvre.

La présente invention a précisément pour but de fournir une méthode de dosage des furocoumarines qui soit plus rapide et plus simple à mettre en oeuvre que les méthodes actuelles et qui soit au moins aussi fiable que ces dernières.

La présente invention a pour objet une méthode de dosage des furocoumarines, et plus particulièrement du psoralène et de ses dérivés tels que le 5-méthoxypsoralène (5-MOP), le 8-MOP et le 5-8-MOP, caractérisée en ce qu'elle comprend une étape de réaction immunologique entre d'une part les furocoumarines susceptibles d'être présentes dans un échantillon biologique, notamment dans le sang prélevé chez un individu, et, d'autre part, des anticorps anti-furocoumarines tels que ceux obtenus par immunisation d'animaux avec des composés constitués de furocoumarines liées à des molécules porteuses antigéniques (ces composés étant encore désignés dans la suite de ce texte par l'expression "complexes furocoumarines/molécules porteuses"), ces anticorps étant susceptibles de reconnaître et de se lier aux furocoumarines à doser, suivie d'une étape de dosage de ces furocoumarines par détection et mesure de la quantité d'anticorps anti-furocoumarines liés à ces dernières.

L'invention vise plus particulièrement une méthode de dosage telle que décrite ci-dessus et caractérisée en ce qu'elle est réalisée à l'aide d'anticorps obtenus par immunisation d'animaux, notamment de lapins, avec des composés eux-mêmes obtenus par couplage d'une molécule porteuse, telle que l'albumine de sérum de boeuf (BSA) ou humaine (HSA), ou l'hemacyanine "keyhole limpet", avec des furocoumarines de formule (I) 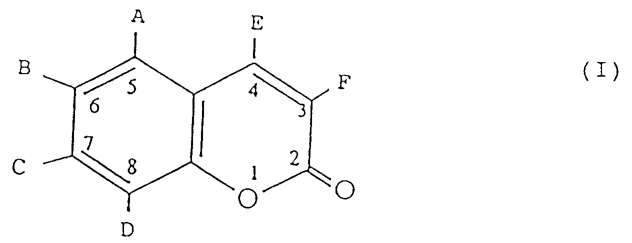
dans laquelle:
- A, B, C, D, E et F, indépendamment les uns des autres, représentent:
   * un groupe de formule -O-(X)ₙ-R, ou (X)ₙ-R dans lequel X représente une chaîne hydrocarbonée comportant 1 à 10, et de préférence 1 à 5 atomes de carbone, le cas échéant substituée, n est égal à zéro ou à 1 et R représente une fonction acide (-COOH), ou amine (-NH₂), ou thiol (-SH),
   * ou un groupe alcoyle comportant 1 à 10, et de préférence 1 à 5 atomes de carbone,
   * ou un atome d'hydrogène,
   * ou un groupement thiol (-SH),
- A et B, ou B et C, ou C et D, ou E et F se branchent avec un cycle de formule (II) 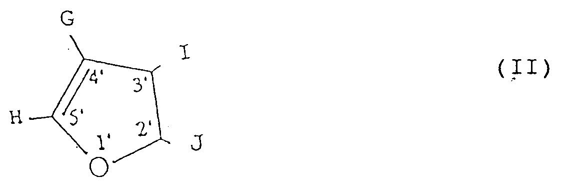 par l'intermédiaire des liaisons G et H, ou I et J, les liaisons G et H, ou I et J qui ne sont pas mobilisées pour le branchement sur le cycle coumarinique de formule (I) ayant la même signification que celle indiquée précédemment pour A, B, C, D, E et F,

l'un au moins de A, B, C, D, E ou F représentant un groupe de formule -O-(X)ₙ-R ou (X)ₙ-R susmentionné, tandis que l'une au moins des associations entre A et B, ou B et C, ou C et D, ou E et F représente un cycle de formule (II) décrit ci-dessus.

Des furocoumarines de formule (I) susmentionnées particulièrement préférées sont choisies parmi:
- l'acide 5-oxyacétique-psoralène (5-OAP) de formule 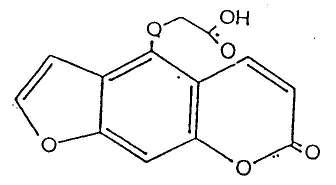
- l'acide 8-oxyacétique-psoralène (8-OAP) de formule 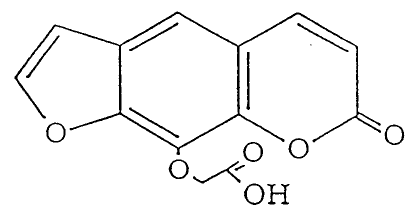
- ou l'acide 5-8-oxyacétique-psoralène (5-8-OAP).

Selon un mode de réalisation particulièrement avantageux de la méthode de dosage de l'invention, cette dernière comprend les étapes suivantes:
- mise en présence de l'échantillon biologique susceptible de contenir les furocoumarines à doser, avec une quantité déterminée d'anticorps anti-furocoumarines, le cas échéant marqués, notamment à l'aide d'enzymes, ou par fluorescence, ou par un radio-isotope, pendant un temps suffisant pour permettre la formation de complexes immunologiques entre les furocoumarines et les anticorps susmentionnés,
- addition du milieu obtenu à l'étape précédente sur un support solide, sur lequel sont fixés des complexes furocoumarines/molécules porteuses susceptibles d'être reconnus par les anticorps susmentionnés, pendant un temps suffisant pour permettre la formation de complexes immunologiques entre les anticorps non liés aux furocoumarines, lors de l'étape précédente de mise en présence de l'échantillon biologique avec lesdits anticorps, et les complexes furocoumarines/molécules porteuses fixés sur le support solide,
- rinçage du support solide,
- détection des anticorps demeurant fixés, après l'étape de rinçage précédente, sur le support solide par l'intermédiaire des complexes furocoumarines/molécules porteuses susmentionnés, soit directement lorsque ces anticorps sont marqués, soit indirectement lorsqu'ils ne sont par marqués, notamment à l'aide d'immunoglobulines susceptibles de reconnaître ces anticorps et étant marquées, notamment à l'aide d'enzymes, ou par fluorescence, ou par un radio-isotope.

Avantageusement le support solide utilisé pour la mise en oeuvre d'une méthode de dosage immuno-enzymatique telle que décrite ci-dessus, est une plaque de microtitration constituée de puits telles que les plaques classiquement utilisées dans les tests ELISA (Enzyme Linked Immuno Sorbent Assay).

L'invention vise également les dérivés furocoumariniques de formule (I) décrits ci-dessus en tant que tels, ainsi que leur procédé d'obtention.

A ce titre, l'invention a également pour objet tout procédé d'obtention des dérivés de formule (I), notamment à partir de dérivés furocoumariniques extraits d'essence de plantes (telle que la bergamote), de formule (Ibis) suivante: 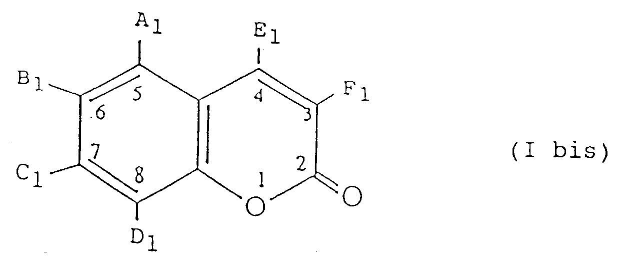
dans laquelle:
- A₁, B₁, C₁, D₁, E₁ et F₁, indépendamment les uns des autres, représentent un atome d'hydrogène ou un groupe OR₁ ou R₁, R₁ représentant une chaîne hydrocarbonée comportant 1 à 10, et de préférence 1 à 5 atomes de carbone, le cas échéant substituée,
- A₁ et B₁, ou B₁ et C₁, ou C₁ et D₁, ou E₁ et F₁ forment en association un cycle de formule (IIbis) 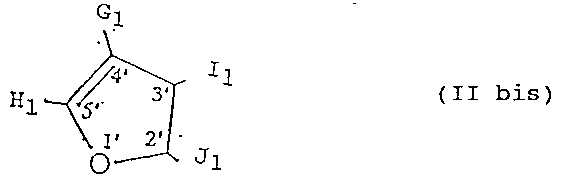 dans laquelle H₁, G₁, I₁ et J₁ ont la même signification que celle indiquée précédemment pour A₁, B₁, C₁, D₁, E₁ ou F₁'
   l'un au moins de A₁, B₁, C₁, D₁, E₁ ou F₁ représentant un groupe de formule -OR₁, ou R₁, tandis que l'une au moins des associations A₁ et B₁, B₁ et C₁, C₁ et D₁ ou E₁ et F₁ représente un cycle de formule (IIbis) décrit ci-dessus,
   lesdits dérivés de formule (Ibis) étant traités par un mélange HCl/pyridine, ce qui conduit à la transformation du (ou des) groupe(s) -OR₁ en groupe -OH, cette étape étant suivie par la mise en présence des composés ainsi obtenus avec des dérivés de formule Y-(X)ₙ-R₂ dans laquelle Y représente un atome d'halogène (notamment du brome ou du chlore), X est tel que défini ci-dessus pour les dérivés de formule (I), n vaut zéro ou 1, R₂ représente -NH₂ ou un groupe -COOR₃ dans lequel R₃ est un groupe alcoyle comportant 1 à 10, et de préférence 1 à 5 atomes de carbone, ce qui conduit aux dérivés de formule (I) dans lesquels R représente -NH₂ lorsque R₂ représente -NH₂ ou aux dérivés de formule (Ibis) dans laquelle -OR₁ ou R₁ est remplacé par le groupe -(X)ₙ-COOR₃ lorsque R₂ représente -COOR₃, ces derniers dérivés étant alors traités par HCl dans un mélange H₂O/EtOH pour conduire aux dérivés de formule (I) dans lesquels R représente -COOH.

L'invention a également pour objet les complexes furocoumarines/molécules porteuses antigéniques tels que définis ci-dessus.

A ce titre l'invention a plus particulièrement pour objet les composés en tant que tels constitués d'un dérivé de formule (I) lié à une molécule porteuse antigénique, notamment à une protéine antigénique telle que la BSA, HSA ou KLH, ainsi que leur procédé d'obtention.

La liaison entre le dérivé de formule (I) et la BSA ou HSA est assurée par formation d'une liaison de type peptidique entre les fonctions carboxyle ou amine des dérivés de formule (I) et les fonctions amine ou carboxyle respectivement de la BSA ou HSA.

A titre illustratif, le couplage des dérivés de formule (I) pour lesquels R représente -COOH, aux protéines porteuses antigéniques est effectué par activation de la (ou des) fonction(s) -COOH du dérivé de formule (I), notamment par liaison au N-hydroxysuccinimide en présence de dicyclohexyl carbodiimide (DCCI) anhydre selon le schéma décrit plus loin, suivie de la liaison proprement dite du dérivé de formule (I) à la protéine porteuse par mise en présence de ces deux composants dans des proportions finales avantageusement de l'ordre d'une molécule porteuse pour environ 100 molécules de dérivé de formule (I), suivie d'une étape de purification des composés ainsi formés, notamment par dialyse.

Le couplage des dérivés de formule (I) pour lesquels R représente -NH₂, aux protéines porteuses est avantageusement réalisé de la manière suivante:
1) Protection des acides aminés dibasiques de la protéine:
   Il existe des méthodes très classiques permettant de protéger les groupements -NH2 libres des protéines.
   On citera pour référence les méthodes qui font intervenir des groupements protecteurs de type butyloxycarbonyle ou benzyloxycarbonyle (C.Toniolo, G.M. Bonora, V. Moretto et M. Bodansky, *Int. j. Pept. Protein Res.,* 1985, 25, 425).
   D'autres méthodes plus récemment proposées font intervenir le groupement cinnamyloxycarbonyle. Dans un milieu reactionnel contenant de l'eau ou du diméthylsulfoxyde aqueux, et en présence de triéthylamine ce composé sus-cité réagit avec la fonction amine d'un aminoacide (H. Kinoshita, K. Inomata, T. Kameda et H. Kotake, *Chem. Letters,* 1985, 515).
2) Activation des groupements - COOH libres de la protéine:
   Sur une protéine porteuse dont on aura préalablement protégé les groupements- NH2 libres selon une des méthodes sus-décrites, l'activation peut être réalisée de façon classique en faisant réagir la protéine, placée dans un tampon phosphate, avec un carbodiimide pour former un ester. La réaction se faisant en milieu aqueux, on choisira pour cela un carbodiimide disposant d'une fonction amine tertiaire, comme le N-(Cyclohexyl)-N'-(*p*-diethylaminocyclohexyl)-carbodiimide, ou d'une amine quaternaire, comme le N-cyclohexyl-N'-[2-morpholinyl-(4)-ethyl]-carbodiimide metho-*p*-toluènesulfonate.
3) Couplage de la furocoumarine avec la protéine activée.
   La protéine dont les fonctions - COOH ont été estérifiées est alors mise à réagir avec la furocoumarine disposant d'une fonction - NH₂. La liaison peptidique se forme alors entre les 2 composants du milieu réactionnel.
4) "Déprotection" de la protéine ( reformation des groupements NH2).
   Là encore, il existe de nombreuses méthodes possibles, pour éliminer les groupements protecteurs des fonctions amines de la protéine porteuse. Les groupements benzoyles peuvent être éliminés en utilisant de l'acide sulfonique trifluorométhane. (H. Yajima, N. Fujii, K. Akaji, M. Sakurai, M. Nomizu, K. Mizuta, M. Aono, M. Moriga, K. Inoue, R. Hosotani, et T. Tobe, *Chem. Pharm. Bull.,* 1985, 33, 3578). On peut également citer une méthode permettant l'élimination des groupements protecteurs formyles, acétyles, et benzoyles: l'amine protégée est mise à réagir avec du di-t-butyl-dicarbonate, en présence de diméthylaminopyridine (L. Grehn, K. Gunnarsson, et U. Ragnarsson, *J. Chem. Soc. Chem. Commun.,* 1985, 1317).

L'invention vise également les anticorps polyclonaux ou monoclonaux tels qu'obtenus par immunisation d'animaux avec une composition telle que décrite ci-dessus, et susceptibles de reconnaître spécifiquement les furocoumarines et de s'y lier, et plus particulièrement le psoralène, le 5-MOP, le 8-MOP et le 5-8-MOP, ces anticorps étant le cas échéant marqués, notamment à l'aide d'enzymes ou par fluorescence, ou par radio-isotopes.

L'invention a également pour objet l'utilisation de complexes furocoumarines/molécules porteuses et d'anticorps tels que décrits ci-dessus pour la mise en oeuvre d'une méthode de dosage de l'invention, et plus particulièrement pour le dosage du 5-MOP et/ou du 8-MOP, notamment dans le cadre du suivi de traitement de patients avec ces composés.

A ce titre l'invention vise plus particulièrement des kits ou trousses de réactifs pour la mise en oeuvre d'une méthode de dosage selon l'invention, et comprenant:
- des complexes furocoumarines/molécules porteuses tels que décrits ci-dessus, avantageusement fixés sur un support solide,
- des anticorps anti-furocoumarines tels que décrits ci-dessus,
- le cas échéant des réactifs marqués permettant la détection des anticorps susmentionnés, dans le cas où ces derniers ne seraient pas marqués, notamment des immunoglobulines susceptibles de reconnaître ces anticorps et marquées à l'aide d'enzymes ou par fluorescence, ou par radio-isotopes.

L'invention sera davantage illustrée à l'aide de l'exemple qui suit de mise en oeuvre d'une méthode de dosage du 5-MOP selon l'invention, ainsi qu'à l'aide des figures suivantes:
- figure 1: schéma de la préparation de l'acide 5-OAP,
- figure 2: diagramme du suivi de la fabrication du complexe 5-OAP/BSA par spectrophotométrie UV (la densité optique (DO) figurant en ordonnée et la longueur d'onde en abscisse)
   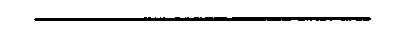 spectre UV BSA-acide 5-OAP
   max: 260,7/206,8
   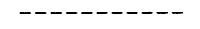 spectre UV BSA
   max: 277,4/220,0
   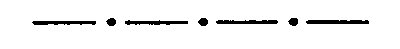 spectre UV acide 5-OAP
   max: 311,6/268,1/249,4
- figure 3: courbes de dosage par la méthode de l'invention du psoralène (carrés pleins) et du 5-MOP (carrés creux) à différentes concentrations, à l'aide d'anticorps anti-5-OAP/BSA; la DO (extinction) à 405 nm est indiquée en ordonnée, la concentration en furocoumarines (ng/ml) étant indiquée en abscisse,
- figure 4: comparaison des courbes de dosage par la méthode de l'invention du psoralène (carrés pleins), du 8-MOP (carrés creux), du 5-8-MOP (losanges pleins), de l'angélicine (losanges creux) et du 5-MOP (triangles pleins) à différentes concentrations, à l'aide d'anticorps anti-5-OAP/BSA; la DO (extinction) à 405 nm est indiquée en ordonnée, la concentration en furocoumarines (ng/ml) étant indiquée en abscisse,
- figure 5: étude de la spécificité des anticorps anti-5-OAP/BSA par comparaison des courbes de dosage du psoralène (carrés pleins), de la coumarine carrés creux), de l'hèrniarine losanges pleins), de l'acide O-coumarique (losanges creux) et de la 4-hydroxycoumarine (triangles pleins).

### I/ Préparation de l'antigène à injecter aux lapins.

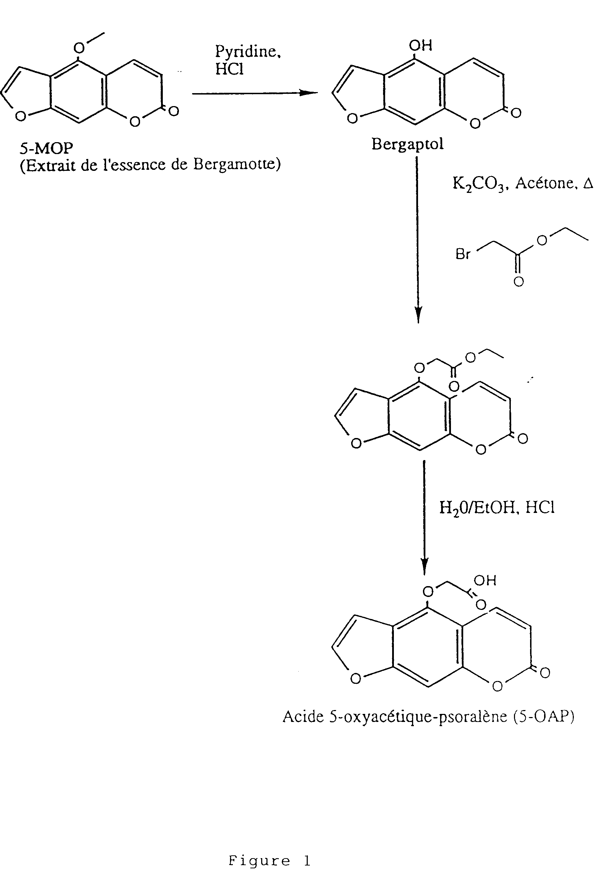

Les furocoumarines ne présentant pas en elles-mêmes de pouvoir antigénique, il a fallu greffer une furocoumarine (haptène) sur une protéine porteuse apportant le pouvoir antigénique (BSA: albumine de sérum bovin).
1/ Préparation de l'haptène
   Le 5-méthoxypsoralène (5-MOP) a été modifié afin de pouvoir le fixer sur les radicaux -NH2 libres de la BSA, par l'intermédiaire d'une liaison peptidique (cf figure n°1). On obtient ainsi l'acide 5-oxyacétique-psoralène (5-OAP) comme haptène.
2/ Couplage de l'haptène (5-OAP) avec la protéine porteuse (BSA).
   Le couplage 5-OAP/BSA s'effectue en réalisant une liaison peptidique, par la méthode N-hydroxysuccinimide (N-HSI)/Dicyclohexylcarbodiimide (DCCI). Il s'agit d'abord d'activer le 5-OAP avant de réaliser la liaison proprement dite.
   a/ Activation du 5-OAP.
      Le solvant de travail est le formaldéhyde. 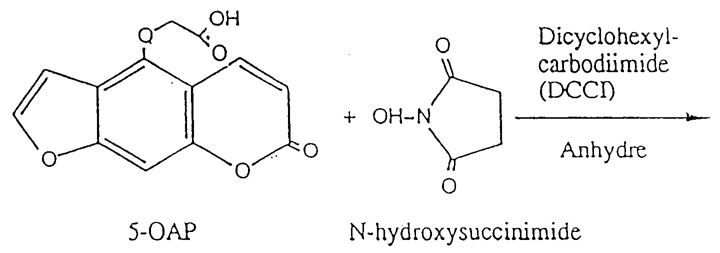 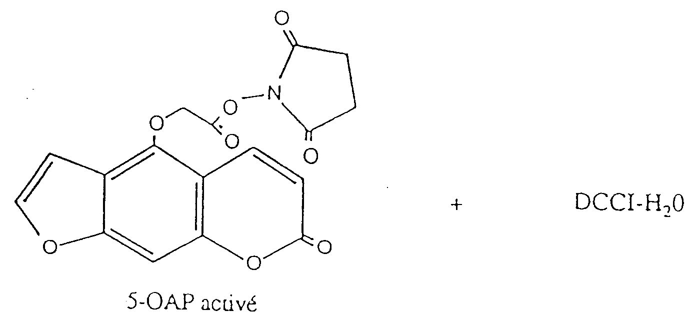
   b/ Liaison du 5-OAP activé avec la BSA.

La BSA est placée dans un tampon phosphate pH = 8,2; on ajoute goutte à goutte le formaldéhyde contenant le 5-OAP activé jusqu'à arriver, environ, à des proportions finales de 1 molécule de BSA pour 120 molécules de 5-OAP activé.

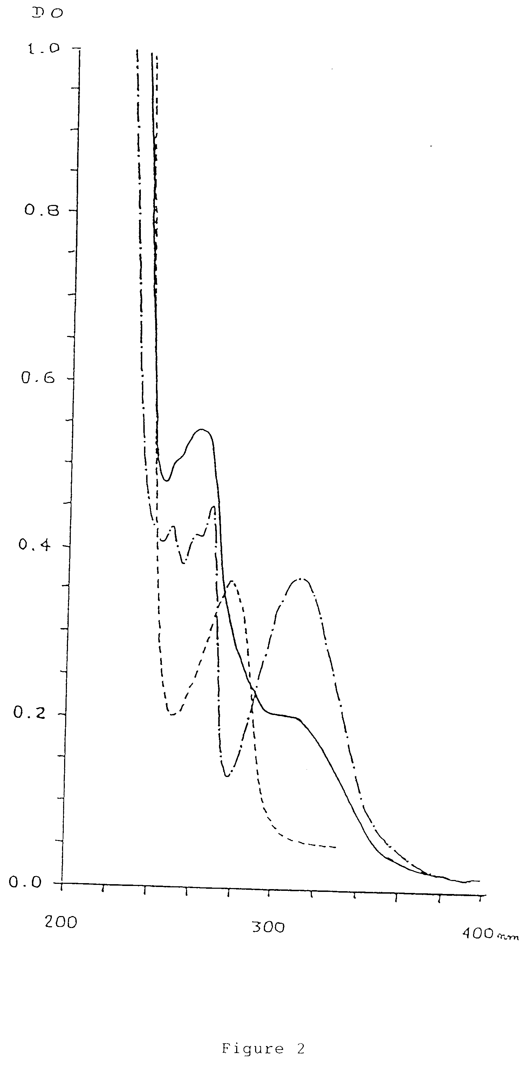

La BSA liée à la furocoumarine est mise à dialyser 24 h dans un Tampon phosphate à pH 8,2, pour éliminer les molécules de 5-OAP activées en excès qui ne se sont pas liées. La pureté du complexe BSA/5-OAP est vérifiée par spectroscopie U.V.(cf Fig. n°2).

Le nombre de 5-OAP fixé par molécule de BSA a été contrôlé en employant un test de dosage des radicaux -NH₂ libres de ce complexe BSA/5-OAP par rapport à une BSA native (test au TNBS). En moyenne, 22 molécules de 5-OAP ont été fixées sur une molécule de BSA.

### II/ Immunisation des lapins.

Trois lapins de laboratoire ont été utilisés, et pour chacun d'entre eux:
- le sérum préimmune a été prélevé 3 jours avant la première injection,
- les injections du complexe BSA/5-OAP ont eu lieu à t=0 jour, t=15 jours, t=30 j, et ainsi de suite de mois en mois. Chaque injection a consisté à injecter 1 mg du conjugué BSA/5-OAP sur le dos du lapin en fractionnant l'apport sur 5 sites différents de la peau (injections sous-cutanées),
- le premier sérum dit "immune" a été prélevé à t=33 jours, puis de mois en mois.

### III/ Dosage des furocoumarines par ELISA.

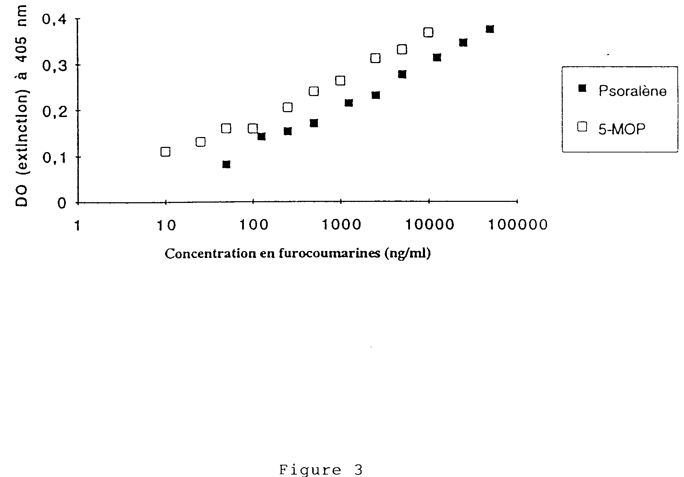

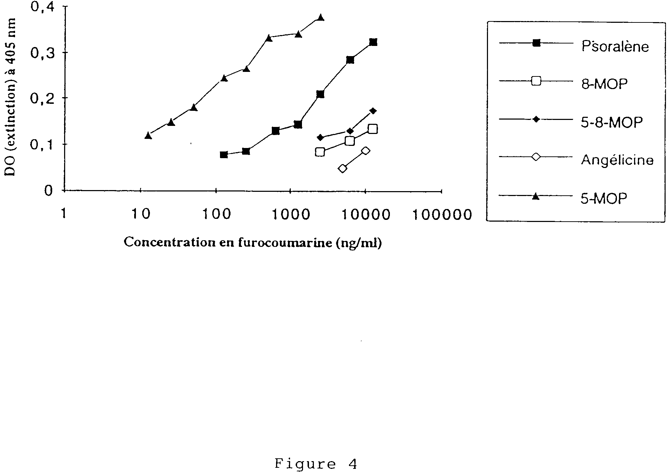

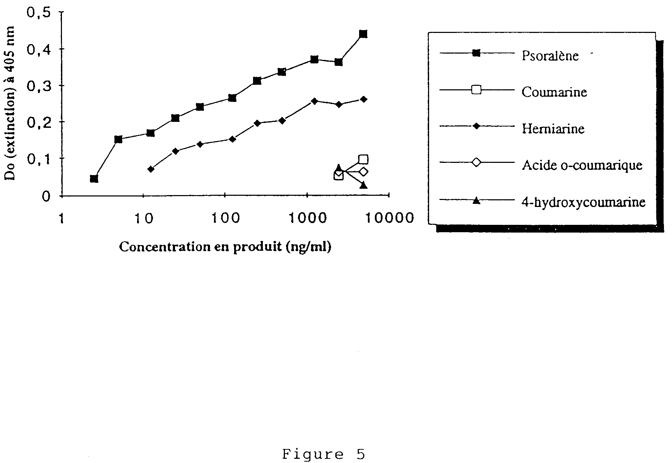

1/ Protocole du dosage des furocoumarines en solution
   Les premières tentatives de dosage des furocoumarines en solution ont été confrontées au problème du non attachement de ces molécules sur les parois des plaques de microtitration. De nombreuses tentatives ont été faites: utilisation de plaques de fabricants différents, traitement des plaques aux UV, utilisation de protéines ou d'ADN/ARN comme agent de liaison entre les parois et les furocoumarines, utilisation de membranes en nitrate de cellulose, etc... Aucune de ces méthodes ne s'est avérée satisfaisante.
   Il a donc été décidé de doser les furocoumarines en solution de manière indirecte par une réaction de compétition.
   Le complexe 5-OAP /BSA se fixe, en effet, facilement sur les plaques par adsorption de sa partie protéique; d'où la possibilité de réaliser une compétition pour la fixation des anticorps de lapin anti-furocoumarine entre le 5-OAP, fixé à une plaque, et le psoralène en solution.
   a/ Sensibilisation des plaques de microtitration.
      On prépare une solution de 5-OAP/BSA à la concentration de 4 µg.ml⁻¹. Cette solution est déposée dans les puits à raison de 50 µl/puits. Chaque plaque est laissée à incuber pendant une nuit à 27°C. Une fois incubées, les plaques peuvent être congelées.
   b/ Lavage des plaques.
      Les plaques sont rincées deux fois avec un tampon phosphate pH= 7,2 (volume de rinçage de 50 µl par puits).
   c/ Réalisation de la réaction de compétition.
      A une solution de furocoumarine de concentration donnée, on ajoute un aliquote de sérum de lapin de manière à obtenir une dilution du sérum au 1/500 ème, ainsi que de la BSA (0,5/1000 p/v). Le temps de contact entre la furocoumarine et le sérum n'influe pas sur le résultat de la compétition.
      Un volume de 50 µl de cette fraction est ajouté dans un puits de la plaque, et on laisse se réaliser la compétition pendant 2 heures à 27°C. Il est recommandé de réaliser 4 répétitions (4 puits) par concentration de furocoumarine.
      Pour chaque plaque, on réalise également une série de 4 témoins correspondant à une solution de sérum diluée au 1/500 ème avec la même concentration de BSA (zéro de concentration en furocoumarines).
   d/ Lavage.
      Même lavage que précédemment.
   e/ Ajout des anticorps de chèvre anti-lapin.
      On prépare une solution avec un sérum d'immunoglobulines de chèvre anti-lapin liées à la phosphatase alcaline, dans un tampon pH = 7,2 avec la BSA à la concentration de 0,5/1000. Le sérum de chèvre est utilisé à la dilution de 1/1000. On ajoute 50 µl de cette solution par puits et on incube 2 h à 27°C.
   f/ Lavage.
      Même protocole que précédemment.
   g/ Ajout du substrat de l'enzyme.
      Le substrat est le paranitrophenylphosphate (PNPP) préparé à raison de 1 mg.ml⁻¹ dans une solution de diéthanolamine 10% p/v à pH= 9,6. Ajouter 50 µl par puits.
   h/ Lecture.
      La lecture s'effectue à 405 nm après 45 mn de réaction de coloration. Il y a possibilité de bloquer la réaction de coloration en ajoutant 50 µl de NaOH 5N par puits.
   i/ Calcul de la valeur d'extinction.
      La différence entre la densité optique du témoin (zéro furocoumarines) et d'un échantillon nous donne la valeur de DO d'extinction (DOₑₓₜ) pour la concentration correspondante en furocoumarines.
2/ Analyse quantitative des furocoumarines en solution.
   On montre expérimentalement que DOₑₓₜ est une fonction linéaire du logarithme de la concentration en furocoumarine (cf Fig. n° 3).
   Les anticorps présentent une très bonne aptitude au dosage du bergaptène (5-MOP) le seuil de détection étant d'environ 10 ng.ml⁻¹ (soit 0,5 ng par puits dans les conditions de l'expérience).
   Le psoralène est également bien reconnu, avec un seuil de détection de 60 ng.ml⁻¹.
   Les autres furocoumarines testées sont moins bien identifiées par les anticorps, et une réponse décroissante est obtenue avec: 5-8-MOP, 8-MOP, angélicine (Fig. n° 4).
   Il est à noter que le psoralène et le 5-MOP avec le cycle lactone ouvert (milieu fortement alcalin) présentent la même droite de calibration qu'avec le cycle lactone fermé pour des concentrations inférieures à 5 µg.ml⁻¹. Les anticorps devraient donc reconnaître les furocoumarines sous forme glycosidée.
   En ce qui concerne les coumarines proprement dites (Fig. n° 5), seule l'hérniarine donne une réponse quantifiable, sans doute attribuable à sa forte analogie structurale avec le psoralène. L'umbelliférone, la coumarine, la 4-hydroxycoumarine et l'acide o-coumarique ne donnent pas des réponses identifiables par rapport au bruit de fond.
3/ Evaluation du titre des différents sérums.
   Les titres de sérums ont été déterminés avec des plaques sensibilisées selon le protocole décrit en 1/ a/. On réalise des dilutions pour chaque sérum prélevé. Le titre du sérum correspond à la dilution qui donne une DO égale à la moitié de la DO maximale obtenue dans l'expérience.
   Les trois lapins ont montré des évolutions comparables en matière de titre de leurs sérums. Un an après l'immunisation, le titre s'élevait à environ 30 000.

## Revendications

1. Méthode de dosage des furocoumarines, et plus particulièrement du psoralène et de ses dérivés tels que le 5-méthoxypsoralène (5-MOP), le 8-MOP et le 5-8-MOP, caractérisée en ce qu'elle comprend une étape de réaction immunologique entre d'une part les furocoumarines susceptibles d'être présentes dans un échantillon biologique, notamment dans le sang prélevé chez un individu, et, d'autre part, des anticorps anti-furocoumarines tels que ceux obtenus par immunisation d'animaux avec des composés constitués de furocoumarines liées à des molécules porteuses antigéniques (ou complexes furocoumarines/molécules porteuses), ces anticorps étant susceptibles de reconnaître et de se lier aux furocoumarines à doser, suivie d'une étape de dosage de ces furocoumarines par détection et mesure de la quantité d'anticorps anti-furocoumarines liés à ces dernières.

2. Méthode de dosage selon la revendication 1, caractérisée en ce qu'elle est réalisée à l'aide d'anticorps obtenus par immunisation d'animaux, notamment de lapins, avec des complexes eux-mêmes obtenus par couplage d'une molécule porteuse, telle que l'albumine de sérum de boeuf (BSA) ou humaine (HSA), ou l'hemacyanine "keyhole limpet" (KLH), avec des furocoumarines de formule (I) 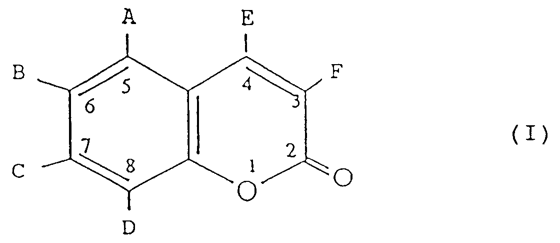 dans laquelle:
- A, B, C, D, E et F, indépendamment les uns des autres, représentent:
* un groupe de formule -O-(X)ₙ-R, ou -(X)ₙ-R dans lequel X représente une chaîne hydrocarbonée comportant 1 à 10, et de préférence 1 à 5 atomes de carbone, le cas échéant substituée, n est égal à zéro ou à 1 et R représente une fonction acide (-COOH), ou amine (-NH₂), ou thiol (-SH),
* ou un groupe alcoyle comportant 1 à 10, et de préférence 1 à 5 atomes de carbone,
* ou un atome d'hydrogène,
* ou un groupement thiol (-SH),
- A et B, ou B et C, ou C et D, ou E et F se branchent avec un cycle de formule (II) 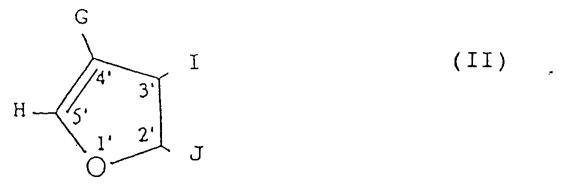 par l'intermédiaire des liaisons G et H, ou I et J, les liaisons G et H, ou I et J qui ne sont pas mobilisées pour le branchement sur le cycle coumarinique de formule (I) ayant la même signification que celle indiquée précédemment pour A, B, C, D, E et F,
l'un au moins de A, B, C, D, E ou F représentant un groupe de formule -O-(X)ₙ-R ou -(X)ₙ-R susmentionné, tandis que l'une au moins des associations entre A et B, ou B et C, ou C et D, ou E et F représente un cycle de formule (II) décrit ci-dessus.

3. Méthode de dosage selon la revendication 1 ou la revendication 2, caractérisée en ce que les furocoumarines de formule (I) sont choisies parmi l'acide S-oxyacétique-psoralène (5-OAP), l'acide 8-OAP, ou l'acide 5-8-OAP.

4. Méthode de dosage selon l'une des revendications 1 à 3, caractérisée en ce qu'elle comprend les étapes suivantes:
- mise en présence de l'échantillon biologique susceptible de contenir les furocoumarines à doser, avec une quantité déterminée d'anticorps anti-furocoumarines, le cas échéant marqués, notamment à l'aide d'enzymes ou par fluorescence, ou par un radio-isotope, pendant un temps suffisant pour permettre la formation de complexes immunologiques entre les furocoumarines et les anticorps susmentionnés,
- addition du milieu obtenu à l'étape précédente sur un support solide, sur lequel sont fixés des complexes furocoumarines/molécules porteuses susceptibles d'être reconnus par les anticorps susmentionnés, pendant un temps suffisant pour permettre la formation de complexes immunologiques entre les anticorps non liés aux furocoumarines, lors de l'étape précédente de mise en présence de l'échantillon biologique avec lesdits anticorps, et les complexes furocoumarines/molécules porteuses fixés sur le support solide,
- rinçage du support solide,
- détection des anticorps demeurant fixés, après l'étape de rinçage précédente, sur le support solide par l'intermédiaire des complexes furocoumarines/molécules porteuses susmentionnés, soit directement lorsque ces anticorps sont marqués, soit indirectement lorsqu'ils ne sont par marqués, notamment à l'aide d'immunoglobulines susceptibles de reconnaître ces anticorps et étant marquées, notamment à l'aide d'enzymes ou par fluorescence ou par un radio-isotope.

5. Dérivés furocoumariniques de formule (I): 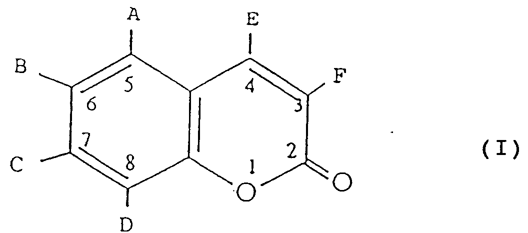 dans laquelle:
- A, B, C, D, E et F, indépendamment les uns des autres, représentent:
* un groupe de formule -O-(X)ₙ-R, ou -(X)ₙ-R dans lequel X représente une chaîne hydrocarbonée comportant 1 à 10, et de préférence 1 à 5 atomes de carbone, le cas échéant substituée, n est égal à zéro ou à 1 et R représente une fonction acide (-COOH), ou amine (-NH₂), ou thiol (-SH),
* ou un groupe alcoyle comportant 1 à 10, et de préférence 1 à 5 atomes de carbone,
* ou un atome d'hydrogène,
* ou un groupement thiol (-SH),
- A et B, ou B et C, ou C et D, ou E et F se branchent avec un cycle de formule (II) 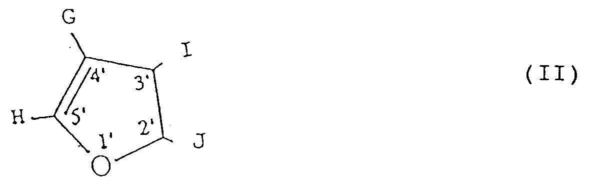 par l'intermédiaire des liaisons G et H, ou I et J, les liaisons G et H, ou I et J qui ne sont pas mobilisées pour le branchement sur le cycle coumarinique de formule (I) ayant la même signification que celle indiquée précédemment pour A, B, C, D, E et F,
l'un au moins de A, B, C, D, E ou F représentant un groupe de formule -O-(X)ₙ-R ou -(X)ₙ-R susmentionné, tandis que l'une au moins des associations entre A et B, ou B et C, ou C et D, ou E et F représente un cycle de formule (II) décrit ci-dessus.

6. Composés, caractérisés en ce qu'ils sont constitués de furocoumarines liées à des molécules porteuses antigéniques, et plus particulièrement d'un dérivé selon la revendication 5 lié à une molécule porteuse antigénique, notamment à la BSA, ou HSA, ou la KLH.

7. Anticorps polyclonaux ou monoclonaux tels qu'obtenus par immunisation d'animaux avec un composé selon la revendication 6, et susceptibles de reconnaître spécifiquement les furocoumarines et de s'y lier, et plus particulièrement le psoralène, le 5-MOP, le 8-MOP et le 5-8-MOP, ces anticorps étant le cas échéant marqués, notamment à l'aide d'enzymes ou par fluorescence, ou par un radio-isotope.

8. Utilisation de composés selon la revendication 6 et d'anticorps selon la revendication 7, pour la mise en oeuvre d'une méthode de dosage selon l'une des revendications 1 à 5, et plus particulièrement pour le dosage du 5-MOP et/ou du 8-MOP, notamment dans le cadre du suivi de traitement de patients avec ces composés.

9. Kit pour la mise en oeuvre d'une méthode de dosage selon l'une des revendications 1 à 5, caractérisé en ce qu'il comprend:
- des complexes furocoumarines/molécules porteuses selon la revendication 6, avantageusement fixés sur un support solide,
- des anticorps anti-furocoumarines selon la revendication 7,
- le cas échéant des réactifs marqués permettant la détection des anticorps susmentionnés, dans le cas où ces derniers ne seraient pas marqués, notamment des immunoglobulines susceptibles de reconnaître ces anticorps et marquées à l'aide d'enzymes ou par fluorescence, ou par un radio-isotope.
